# EUROPEAN PATENT APPLICATION

(11) **EP 1 374 934 A1**
(43) Date of publication of application: **02.01.2004**
(21) Application number: 02425422.9
(22) Date of filing: 25.06.2002
(51) Int. Cl.: A61M 5/32

(54) **Improved safety butterfly needle**

(71) Applicant: Restelli, Sergio, 00100 Rome (IT); Righi, Nardino, 20047 Brugherio (Milano) (IT); Rossi, Roberto, 20151 Milano (IT)
(72) Inventor: Restelli, Sergio, 00100 Rome (IT); Righi, Nardino, 20047 Brugherio (Milano) (IT); Rossi, Roberto, 20151 Milano (IT)
(74) Representative: Petruzziello, Aldo

(57) **Abstract**

A butterfly needle (100) for venipuncture comprises a body (1) with two protruding flexible tongues (2) that can be bent by manual gripping by the user from a diverging position to a converging position, and a needle (9) mounted axially in the fore end of a needle-carrier (3) which has a tail (8) protruding rearward from the body (1) to couple with the connection of a flexible tube destined to be connected to a syringe or a bottle. The needle-carrier (3) is mounted axially slidable in the body (1) to pass from a forward position of use wherein the needle (9) protrudes forward from the body (1) to perform the injection to a retracted position of safety wherein the needle (9) is protected inside the body (1), there being provided guide means (13, 33) able to guide the axial movement of the needle-carrier (3) inside the body (1) and end-of-stroke and locking means (18, 17, 36) able to lock the needle-carrier in said advanced position and in said retracted position.

## Description

The present invention refers to a safety butterfly needle for venipuncture.

As is known, butterfly needles for venipuncture are widely available on the market. Said butterfly needles are generally used for intravenous injections, infusions, blood sampling, transfusions and the like. Although specific reference will be made herein to injections, it is to be understood that the butterfly needle according to the invention can also be used for blood sampling and the like.

A butterfly needle generally comprises a needle proper to penetrate into the vein. Said needle is supported integrally by a needle-carrier whereon two flexible tongues shaped like butterfly wings are mounted.

The rear end of the needle is connected to a small flexible plastic tube. The tube ends in a wider mouth, which is closed by a stopper. The mouth of the tube is designed to couple with the fore end of a syringe filled with a solution to be injected for intravenous injections or with the top of a container or bottle for intravenous infusions.

Said flexible tongues perform a dual function: that is to say, they act as a grip for the operator who must perform the injection and they act as a means of fixing to the patient's skin to secure the needle in place during the injection. Once the treatment has been completed, the user bends the tongues to make them converge and removes the needle from the vein.

The assembly of butterfly needle, tube, syringe, bottle etc. cannot be re-used and therefore is sent for disposal. This operation proves extremely dangerous since the butterfly needle remains exposed and this leads to the risk of accidental needle sticks both by the user performing the injection and by the personnel responsible for disposal.

The object of the present invention is to overcome the drawbacks of the prior art by providing a safety butterfly needle that is able to avoid accidental needle sticks.

Another object of the present invention is to provide such a safety butterfly needle that is practical, simple for the user to use and reliable during use.

Yet another object of the invention is to provide such a safety butterfly needle that is economical and simple to make.

These objects are achieved in accordance with the invention with the characteristics listed in appended independent claim 1.

Advantageous embodiments of the invention are apparent from the dependent claims.

The safety butterfly needle for venipuncture according to the invention comprises a substantially cylindrical body with two outwardly protruding flexible tongues which can be manually gripped by the user and bent from a diverging to a converging position.

A needle is axially mounted in the head of a needle-carrier coupled to the body, so that the needle protrudes axially forward from the body. The needle carrier has a tail protruding rearward from the body to couple with the connection of a flexible tube destined to be connected to a syringe or to a bottle for injection of a solution or for collection of blood.

The main characteristic of the invention lies in the fact that said needle-carrier is mounted so that it can slide axially inside the body to pass from a forward position of use in which the needle protrudes forward from the body to perform the injection and a retracted position of safety in which the needle is protected inside the body. The butterfly needle according to the invention further comprises guide means able to guide the axial movement of the needle-carrier inside the body and end of stroke and locking means able to lock the needle-carrier in said forward position of use and in said retracted position of safety.

The advantages of the butterfly needle with needle-cover device according to the invention are obvious. In fact, once the injection has been performed, the needle is protected by the body in a position of safety, thus avoiding accidental injuries.

Moreover, operation of said butterfly needle appears to be extremely safe, precisely because the needle-carrier is guided during its axial movement within the body.

Further characteristics of the invention will be made clearer by the detailed description that follows, referring to a purely exemplary and therefore non-limiting embodiment thereof, illustrated in the appended drawings, in which:
Figure 1 is an exploded top view illustrating a butterfly needle according to the invention, wherein the front part of the body is shown partially in section;
Figure 2 is a perspective view illustrating the needle-carrier of the butterfly needle according to the invention;
Figure 3 is a plan view from above of the butterfly needle of Figure 1 assembled and in the position of use, wherein the needle cap is not shown and the front part of the body is shown partially in section;
Figure 4 is a cross section of the butterfly needle, taken along the sectional plane IV-IV of Figure 3, wherein the needle-carrier has not been sectioned;
Figure 5 is a plan view from above of the butterfly needle in the safety position wherein the body is shown partially in section;
Figure 6 is a plan view from above illustrating the rear part of the body of the butterfly needle according to a variant embodiment of the invention;
Figure 7 is a plan view from above partially illustrating the needle-carrier and the rear part of the body according to the variant of Figure 6, wherein the needle-carrier is locked in the safety position.

The butterfly needle according to the invention, denoted as whole with reference numeral 100, is described with the aid of the figures.

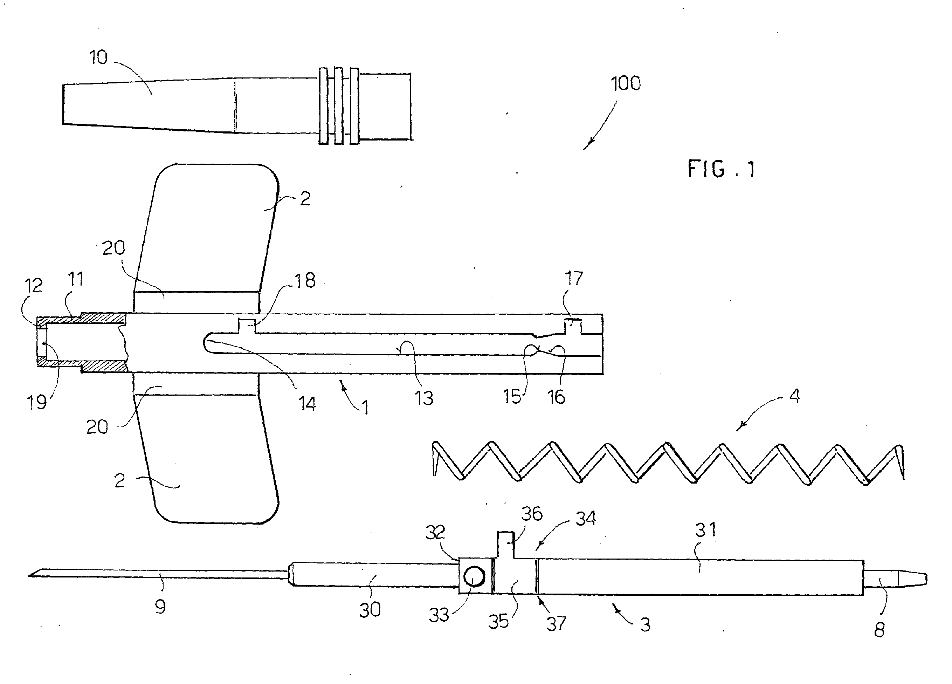

With reference for now to Figure 1, the butterfly needle 100 comprises a main body 1, substantially cylindrical in shape, whereon two flexible tongues 2 are mounted. The tongues 2 are mounted in the front part of the body 1 and protrude in opposite directions with respect to the axis of the body. Near the body 1 the tongues 2 have longitudinal grooves 20 which act as fold lines to allow the tongues 2 to be folded along the grooves 20. In this manner the tongues 2, bending along the fold lines 20, can pass from an open position, shown in the figures, to a closed position, in which they converge, coming close to each other so that they can be grasped by the user.

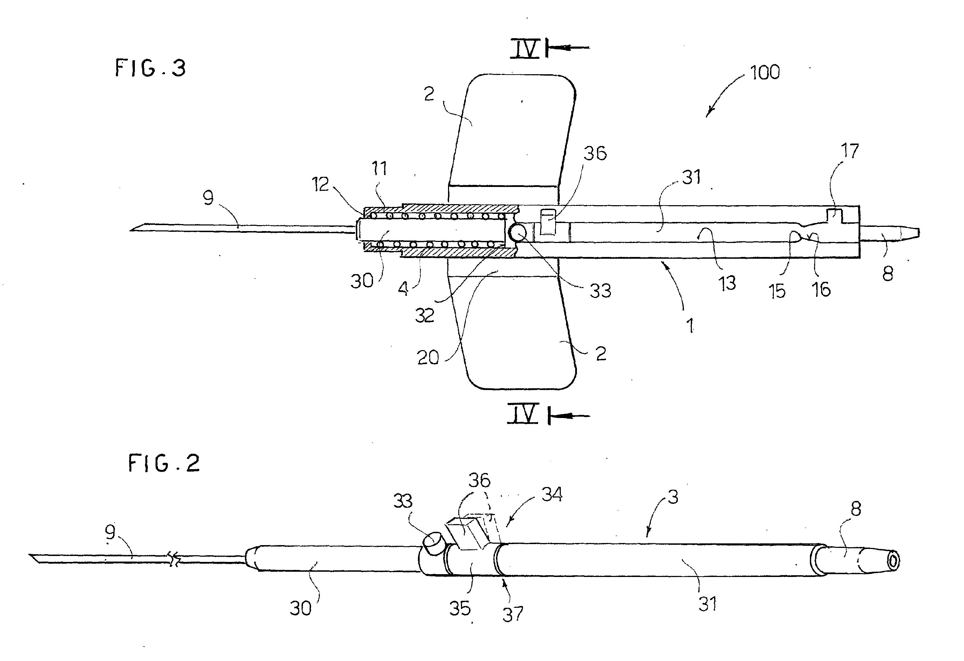

With reference also to Figure 2, a needle 9 is mounted axially in the fore end of a needle-carrier 3. The needle-carrier 3 is hollow on the inside and has an axial channel open at the front and rear for passage of the liquid to be injected. The needle-carrier 3 must be mounted inside the body 1 so that the needle 9 protrudes forward from the body 1 to perform the injection. The needle 9 is covered by a substantially frusto-conical needle cap 10 that snap engages in the fore end 11 of the body 1. For this purpose the fore-end 11 of the body 1 has a slightly smaller diameter than the body 1.

The needle-carrier 3 has a tail 8 designed to protrude rearward from the body 1 to couple with a special connection of a flexible tube (not shown in the figures) designed to be connected to a syringe or to a bottle containing the solution to be injected or destined to receive the blood sampled. The needle-carrier 3 has a front part 30 with a smaller diameter and a central part 31 with a larger diameter, so as to define an annular abutment surface 32 between the front part 30 and the central part 31.

A pin or nib 33, substantially cylindrical in shape and protruding radially outward from the central part 31 of the needle-carrier, is provided in the front in the central part 31 of the needle-carrier.

A manual operating device 34 is mounted behind the nib 33, in the central part 31 of the needle-carrier. The manual operating device 34 comprises a small hollow cylinder or tube 35 rotatably mounted on a recessed seat 37 of the central part 31 of the needle-carrier, so as to be able to turn with respect to the axis of the needle-carrier. In this manner the outer side surface of the rotatable cylinder 35 is at the same level as the outer side surface of the central part 31 of the needle-carrier.

A tongue or operating lever 36 having a substantially parallelepiped shape, suitable to be manually operated be the user's finger tip, protrudes radially outward from the rotatable cylinder. The thickness of the operating lever 36 is slightly less than the diameter of the nib 33.

The body 1 is substantially cylindrical in shape and hollow on the inside, having a bore 11 open at the front and rear. The body 1 has a longitudinal slot 13 in its side surface. The longitudinal slot 13 is disposed at an angular distance of about 90° with respect to the wings 2. The longitudinal slot 13 is delimited at the front by a rounded front abutment surface 14. The width of the longitudinal slot 13 is slightly greater than the diameter of the nib 33.

Behind the front abutment surface 14, the slot 13 has a recess 18 acting as a front stopping seat, disposed circumferentially at right angles with respect to the axis of the longitudinal slot 13. The width of the front recess 18 is slightly greater than the width of the operating lever 36 to be able to receive it on its inside. In practice the slot 13 has a substantially L-shaped configuration at the front to allow a bayonet-type coupling.

At the rear, on the other hand, the longitudinal slot 13 has a narrowing 15. The width of the narrowing 15 is smaller than the diameter of the nib 33 and larger than the thickness of the operating tongue 36, so as to allow the passage of the tongue 36 and locking of the nib 33. Behind the narrowing 15, the longitudinal slot 13 has a tapered part 16 which widens. Behind the tapered part 16 a recess 17 acting as a rear stopping seat, able to receive the operating lever 36, is provided. The rear recess 17 is disposed circumferentially at right angles with respect to the axis of the longitudinal slot 13. In practice, the slot 13 has at the rear also a substantially L-shaped configuration to allow a bayonet-type coupling.

The head 11 of the body 1 has in its front edge an annular collar 12 protruding inward so as to form a radial abutment surface and define a hole 19 able to allow the passage of the front part 30 of the needle-carrier 3.

The butterfly needle 100 according to the invention comprises a spiral spring 4 suitable to be disposed around the front part 30 of the needle-carrier 3, so that one end of the spring 4 abuts against the abutment surface 32 of the central part 31 of the needle-carrier 3 and the other end of the spring 4 abuts against the collar 12 of the head 11 of the body 1.

Assembly and operation of the butterfly needle 100 will now be described purely by way of example.

The spring 4 is inserted around the front part 30 of the needle-carrier 3, then the needle-carrier 3 with the spring 4 is inserted from the rear into the body 1, so that the spring 4 is compressed between the collar 12 of the fore end of the body 1 and the abutment surface 32 of the needle-carrier 3. During this operation of insertion, the lever 36 of the manual operating device is aligned with the nib 33 of the needle-carrier and both are disposed in the slot 13. Then the needle-carrier 3 is guided it its forward stroke inside the body 1.

When the needle-carrier 3 reaches the forward end of stroke, the nib 33 of the needle-carrier abuts against the front abutment surface 14 of the slot 13, then the manual operating device is operated in rotation, so that the lever 36 engages in the front recess 18.

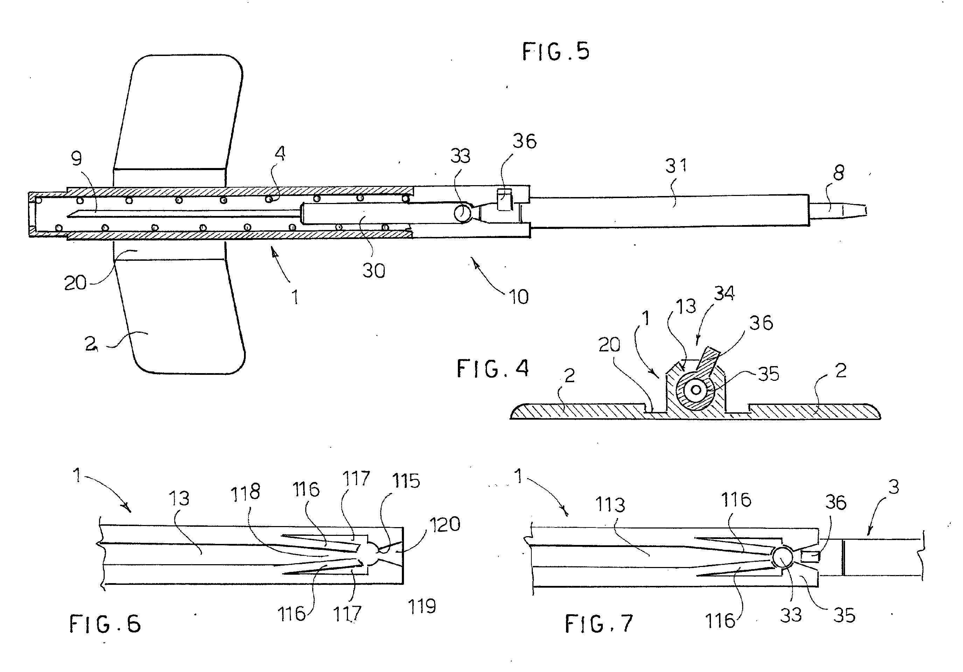

As shown in Figures 3 and 4, in this situation the spring 4 is loaded and compressed between the collar 12 of the body and the abutment surface 32 of the needle carrier and the needle-carrier is locked in axial movement since the lever 36 of the manual operating device is inside the front recess 18. Furthermore, the front end of the front part 30 of the needle-carrier protrudes from the hole 19 of the head of the body and the needle 9 protrudes forward and axially out of the body 1.

Once the injection has been performed, the user turns the manual operating device 34, so as to disengage the lever 36 from the locking recess 18 and position it in axis with the slot 13. As a result, the needle-carrier is no longer retained and the spring 4 is released, causing retraction of the needle-carrier 3 with respect to the body 1.

As shown in Figure 5, at the end of the retraction stroke of the needle carrier, the nib 33 abuts against the narrowing 15 of the slot 13, thus preventing further retraction of the needle-carrier 3 or possible extraction thereof from the body 1. At this point, the user rotates the manual operating device turning the lever 36, so that the lever 36 engages in the rear recess 17. In this situation any axial movement of the needle-carrier 3 with respect to the body 1 is prevented and the needle 9 remains in the safety position, protected inside the body 1.

As shown in Figures 6 and 7, to avoid operation of the manual operating device, when the needle-carrier is in the rearward end of stroke point, a different shape of the rear part of the body 1 can be foreseen. In this case, two oblique flexible tongues 116 disposed in the slot 13 are provided in the rear part of the body 1, so as to define a tapered part 118 that narrows. The tongues 116 are obtained by means of longitudinal cuts 117 on the body 1.

Behind the tongues 116 a narrowing 115, narrower than the diameter of the nib 33 and wider than the thickness of the operating lever 36, is provided. The end surfaces 119 of the tongues 116 are disposed opposite the narrowing 115. Behind the narrowing 115 a tapered part 120 that widens is provided.

As a result, during retraction of the needle-carrier 3, the tongues 116 of the body diverge to allow the passage of the lever 36 and the nib 33 of the needle-carrier, thus when the nib 33 abuts on the narrowing 115, the tongues 116 return elastically to the original position, so that the ends 119 of the tongues 116 block the nib 33 in the narrowing 115. The operating lever 36, on the other hand, passes the narrowing 115 and is disposed in the tapered rear part 120.

Numerous changes and modifications of detail within the reach of a person skilled in the art can be made to the present invention without departing from the scope of the invention as set forth in the appended claims.

## Claims

1. A butterfly needle (100) for venipuncture comprising
- a substantially cylindrical body (1) with two outwardly extending flexible tongues (2) that can be manually grasped and bent by the user from a diverging position to a converging position, and
- a needle (9) mounted axially in the head of a needle-carrier (3) coupled to said body (1) so that the needle (9) protrudes forward from said body (1), said needle-carrier having a tail (8) protruding rearward from said body (1) to couple with the connection of a flexible tube intended to be connected to a syringe or bottle,
**characterised in that**
said needle-carrier device (3) is mounted so as to slide axially inside the body (1) to pass from a forward position of use wherein the needle (9) protrudes forward from said body (1) to perform the injection to a retracted position of safety in which the needle (9) is protected inside said body (1), there being provided:
- guide means (13, 33) able to guide the axial movement of said needle-carrier (3) inside said body (1) and
- end-of-stroke and locking means (18, 17, 36) able to lock said needle-carrier in said forward position and in said retracted position.

2. A butterfly needle (100) according to claim 1, **characterised in that** spring means (4) are disposed inside said body (1), between a collar (12) of said body (1) and an abutment surface (32) of said needle-carrier (3), said spring means (4) being loaded when said needle-carrier is in the forward position of use.

3. A butterfly needle (100) according to claim 2, **characterised in that** said spring means comprise a spiral spring (4) disposed around a front part (30) of said needle-carrier, having a smaller diameter than a central part (31) of said needle-carrier.

4. A butterfly needle (100) according to any one of the preceding claims, **characterised in that** said guide means comprise a longitudinal slot (13) formed in the body (1) and a substantially cylindrical nib (33) which protrudes radially outward from the needle-carrier (3) to be able to slide within said longitudinal slot.

5. A butterfly needle (100) according to claim 4, **characterised in that** said longitudinal slot (13) is defined by a front abutment surface (14) and a rear narrowing (15; 115) able to block said nib (33) when the needle-carrier (3) is in the forward position of use and the retracted position of safety, respectively.

6. A butterfly needle (100) according to claim 4 or 5, **characterised in that** said end-of-stroke and locking means comprise a manual operating device (34) comprising a hollow cylinder (35) rotatably mounted on said needle-carrier (3) and an operating lever (36) protruding radially from said cylinder (35) and protruding outward from said longitudinal slot (13) to be able to be operated manually by the user, so as to lock said needle-carrier, when it is respectively in the forward position of use and in the retracted position of safety.

7. A butterfly needle (100) according to claim 6, **characterised in that** said longitudinal slot (13) has a front recess (18) at the front, suitable to receive said manual operating lever (36) to lock the needle-carrier in the forward position of use and a rear recess (17) able to receive said manual operating lever (36) to lock the needle-carrier (3) in the retracted safety position.

8. A butterfly needle (100) according to claim 7, **characterised in that** said manual operating device (34) is disposed behind said nib (33), said operating lever (36) has a smaller thickness than said narrowing (15) and said rear locking recess (17) is disposed behind said narrowing (15).

9. A butterfly needle (100) according to any one of claims 5 to 8, **characterised in that** said body (1) comprises at least one flexible tongue (116) disposed obliquely in the rear part of said longitudinal slot (13) so that the free end (119) of said tongue is disposed opposite said narrowing (115) to lock said nib (33) of the needle-carrier in abutment between the narrowing (115) and the free end (119) of the flexible tongue, when the needle-carrier is in the retracted position of safety.

10. A butterfly needle (100) according to claim 9, **characterised in** the said at least one flexible longitudinal tongue (116) is formed by means of a longitudinal cut (117) on said body (1).
